# EUROPEAN PATENT APPLICATION

(11) **EP 4 230 118 A1**
(43) Date of publication of application: **23.08.2023**
(21) Application number: 22157458.5
(22) Date of filing: 18.02.2022
(51) Int. Cl.: A61B 5/00, A61B 5/026, A61B 5/0295

(54) **A SYSTEM AND METHOD FOR TISSUE ANALYSIS USING REMOTE PPG**

(71) Applicant: Koninklijke Philips N.V., 5656 AG Eindhoven (NL)
(72) Inventor: LAI, Marco, Eindhoven (NL); HENDRIKS, Bernardus Hendrikus Wilhelmus, Eindhoven (NL)
(74) Representative: Philips Intellectual Property & Standards

(57) **Abstract**

A PPG imaging system and method processes images to derive remote PPG signals. Images of a region of interest are received, and landmarks are identified in each of a set of the images. For pairs of images of the set, the landmarks between first and second images of the pair are paired, and the second image is deformed to match the landmarks in that second image with the landmarks in the first image thereby providing motion compensation. A remote PPG signal is obtained from a set of the motion-compensated images.

## Description

### FIELD OF THE INVENTION

This invention relates to the analysis of tissue using photoplethysmography (PPG) analysis.

### BACKGROUND OF THE INVENTION

Microcirculation is the circulation of the blood in the smallest blood vessels, present in the vasculature embedded within organ tissues. The structure of the microvasculature is extremely complex, as so many capillaries are present in the tissues, with the individual capillaries so convoluted that the blood flow at any given point in any given capillary could be in any direction. For this reason, it can be stated that their overall function becomes averaged. That is, there is an average rate of blood flow through each tissue capillary bed, an average capillary pressure within the capillaries, and an average rate of transfer of substances between the blood of the capillaries and the surrounding interstitial fluid. This is the perfusion of the tissue.

Organ perfusion is a crucial indicator of injury and disease, which may include inflammation, stagnant or stopped blood flow, and all pathologies that can lead to global tissue hypoxia and organ dysfunction. Perfusion monitoring can be used to assess these microvascular injuries and many others, such as the progress of healing of either burned skin or wounds or the recovery of the perfusion downstream of a vessel lesion, and the necrosis (e.g., foot ulceration, sepsis) for patients with diabetes.

Non-contact PPG imaging is a recent emerging technology able of monitoring skin perfusion. PPG imaging utilizes an off-the-shelf camera and a light source to remotely detect the dynamic changes in blood volume beneath the skin and allows extracting blood pulsation signals. PPG imaging allows for the elaboration of large tissue areas, thus building a so-called perfusion map, which is a great advantage with respect to contact PPG. PPG imaging has shown to be capable of detecting skin perfusion perturbations, such as irritation, temperature changes and even flow blockage during pressure measurements and has even been evaluated for peripheral arterial disease assessment.

It is possible to build an amplitude map, that represents the amplitude of the PPG signal per image sensing pixel. Furthermore, it is also possible to build a delay map, which provides a measure of the average time delay between the PPG signal wave of each pixel and a reference PPG signal. There is a small delay in the blood pulsation arrival, due to small differences in the microcirculatory bed, such as the resistance and elasticity of the vessels, as well as different artery branches that supply the recorded tissues.

For the purposes of this application, which relates to processing of non-contact PPG signals, perfusion may be defined as the amplitude of a PPG signal of the tissue, namely the amplitude of the pulsatility extracted from the tissue.

In order to extract a reliable PPG signal and perfusion map, it is important that each pixel of the image looks at the same small region of the skin. Thus, instead of looking at a video as a series of frames acquired by the camera one after the other, the video images can be considered as a matrix of light sensors that detect the variation in light in small regions of the skin, stored in the pixels of the video.

Motion stabilization is needed for compensating for motion. Usually stabilization is needed when a camera is not still, for example if an operator is holding it or if a video is recorded on a not stable support. Also, it is not possible for the subject to stay completely still during the measurements, some motions will be always present and as a result the sensors of the camera will not always look at the same small regions of investigation.

This issue can be solved by implementing a correction via motion stabilization software. Once video is acquired, the motion stabilization algorithm will stabilize the region of investigation so that in a given pixel there will always be the signal of the same small region of skin.

Unfortunately, in some situations, such us when endoscopic images are acquired inside the body, motion stabilization on the image becomes extremely difficult. Organs and internal body areas in general undergo deformation, and operators cannot hold the endoscope in a fixed situation for long, causing perspective changes. Because of these factors, motion compensation is very hard in these situations.

There is therefore a need for improved motion compensation when collecting remote PPG signals.

### SUMMARY OF THE INVENTION

The invention is defined by the claims.

According to examples in accordance with an aspect of the invention, there is provided a PPG imaging system for processing images to derive remote PPG signals, comprising a processor adapted to:
receive images of a region of interest;
identify landmarks in each of a set of the images;
for pairs of images of the set:
   pair the landmarks between a first image of the pair and a second image of pair; and
   deform said second image of the pair to match the landmarks in that second image of the pair with the landmarks in the first image of the pair thereby providing motion compensation between said pair of images; and
   derive a remote PPG signal from a set of the motion-compensated images.

This system makes use of remote PPG sensing to derive a PPG signal, for example a set of PPG signals which together define a PPG perfusion map and/or a PPG delay map.

The remote PPG signals are obtained by analyzing image changes, for example changes in color over time, at specific locations of the region of interest. The images thus need to be aligned with each other to be able to track the images of specific locations. This alignment is particularly difficult in some situations, for example when the images are obtained using an endoscope. Motion compensation is needed to compensate for the relative movement between the camera and the region of interest. This can be caused by movement of the camera, movement of the region of interest or both.

The motion compensation is achieved by deforming each image such that landmarks in the image are matched with landmarks in a previous image. Thus, over time, a motion-compensated, i.e. motion-stabilized, set of images (i.e. video) is obtained. The remote PPG signals are derived from these motion-compensated images, i.e. from a motion stabilized video. In this way, sequences of images (e.g. video) stabilized by using the described deformation method, can be utilized to extract PPG information. The landmarks are used to stabilize the images, but PPG information can be extracted from the entire stabilized image.

The set of the motion-compensated images for example cover a time period including a heartbeat, for example a video over a 2 second time window. One image may be motion-compensated with an immediately preceding image (so that each image is deformed to match the previous image), or one image may be motion-compensated to an initial image of the image sequence, functioning as a reference image. Thus, the pairs may be adjacent along the time sequence or they may be non-adjacent.

There may be a set of remote PPG signals obtained from different image locations within the set of motion-compensated images.

The processor is for example adapted to derive a set of remote PPG signals comprising a PPG perfusion map based on PPG amplitude levels obtained from the motion-compensated images at a set of image locations.

The processor may additionally or alternatively be adapted to derive a set of remote PPG signals comprising a PPG delay map based on PPG relative delays between image locations.

The delay information enables different tissue types to be identified, either automatically or by user-selection after displaying a suitable representation of the PPG delay map. Different tissue regions for example have different delay times. This may arise because the different tissue types are supplied by different arteries and hence have a different path and hence path length to the heart. This may arise in the case of different portions of the intestines, and these portions may become adjacent during open bowel resection. There is a resulting intestinal anastomosis with communication between formerly distant portions of the intestine.

It is then of interest to analyze the perfusion in these portions separately as this gives a more accurate tissue assessment, as it takes account of the different tissue types and their possible different blood supplies.

The images for example comprise 2D camera images or they may comprise hyperspectral camera images.

The landmarks for example comprise a pattern of a microvasculature visible at the surface of the region of interest. This pattern of microvasculature is for example encoded as branch points and/or vessel lengths. The landmarks may additionally or alternatively comprise applied surgical features such as staples or sutures.

The processor is for example adapted to deform the second image of the pair by using an algorithm that combines image registration and deformation. Examples of such algorithms are a Free Form Deformation algorithm or a Thin Plate Spines algorithm.

The system for example further comprises a 2D camera for capturing the images of the region of interest. The system may further comprise an endoscope, wherein the 2D camera is mounted at a distal end of the endoscope or at the proximal end (near the eyepiece).

The invention also provides a method for processing images to derive remote PPG signals, comprising:
receiving images of a region of interest;
identifying landmarks in each of a set of the images;
for pairs of the images of the set:
   pairing the landmarks between a first image of the pair and a second image of the pair; and
   deforming said second image of the pair to match the landmarks in that second image of the pair with the landmarks in the first image of the pair thereby providing motion compensation between said first and second images; and
   deriving a remote PPG signal from a set of the motion-compensated images.

The method may comprise:
deriving a set of remote PPG signals as a PPG perfusion map based on PPG amplitude levels obtained from the images at a set of image locations; and/or
deriving a set of remote PPG signals as a PPG delay map based on PPG relative delays between image locations.

The invention also provides a computer program comprising computer program code means which is adapted, when said program is run on a computer, to implement the method defined above. The invention also provides a processor which is programed with the computer program

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a representation of a frame of a video acquired of the intestine and a global PPG signal derived from the video;
Figure 2 shows separate PPG signals for separate regions of interest;
Figure 3 shows a PPG perfusion map;
Figure 4 shows a zoom-in of the PPG signals extracted from the two regions of interest from Figure 2 and a PPG delay map;
Figure 5 illustrates a projective transformation;
Figure 6 illustrates an affine transformation for scaling, skewing and rotation;
Figure 7 represents a registration approach with deformation using a Thin Plate Spline algorithm;
Figure 8 shows a conventional approach for extracting PPG signals from pixels of a 2D endoscope and building a PPG perfusion map;
Figure 9 shows the motion stabilization approach to be employed within the general method of Figure 8, in accordance with the invention;
Figure 10 shows an example of landmarks in an endoscopic video frame;
Figure 11 shows system for tissue analysis; and
Figure 12 shows a hypercube of a hyperspectral camera;

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a PPG imaging system and method which processes images to derive remote PPG signals. Images of a region of interest are received, and landmarks are identified in each of a set of the images. For pairs of images of the set, the landmarks between each current image and each next image are paired, and a second image of the pair is deformed to match the landmarks in that second image with the landmarks in the first image thereby providing motion compensation. A remote PPG signal is obtained from a set of the motion-compensated images.

Before describing the system and method of the invention, the known operation of a remote PPG imaging system, and the known image processing methods, will first be described.

Remote PPG imaging enables a determination of tissue perfusion from images captured of the tissue of interest, e.g. the skin or even tissue beneath the skin. Remote PPG typically uses ambient light, functioning as broad band white light source, and the diffuse and specular reflections are analyzed for different color components. Remote PPG imaging may be used to construct a PPG amplitude map and a PPG delay map.

For this purpose, a camera or a series of cameras (at one or multiple wavelengths) captures video of the tissue area, e.g. skin, at a distance. The measurements derived from the video are remote PPG images, which provide non-contact measurement of a pulse signal by analyzing subtle changes of skin color (or organ color) i.e. at different wavelengths of the light.

It has been proposed to use remote PPG for inflammation detection. It has also been proposed in European Patent Application No. 20214955.5 to measure perfusion based both on a PPG amplitude level and also information about the distribution of the PPG phases, such as a standard deviation or interquartile range of a phase map.

By extracting pulse signals at each individual location of the skin region (corresponding to each pixel of the cameras) a spatial map of the pulse signal can be derived, showing both amplitude and delay. This perfusion map thus represents the amplitude and delay of the PPG signal per pixel and hence per location of the skin.

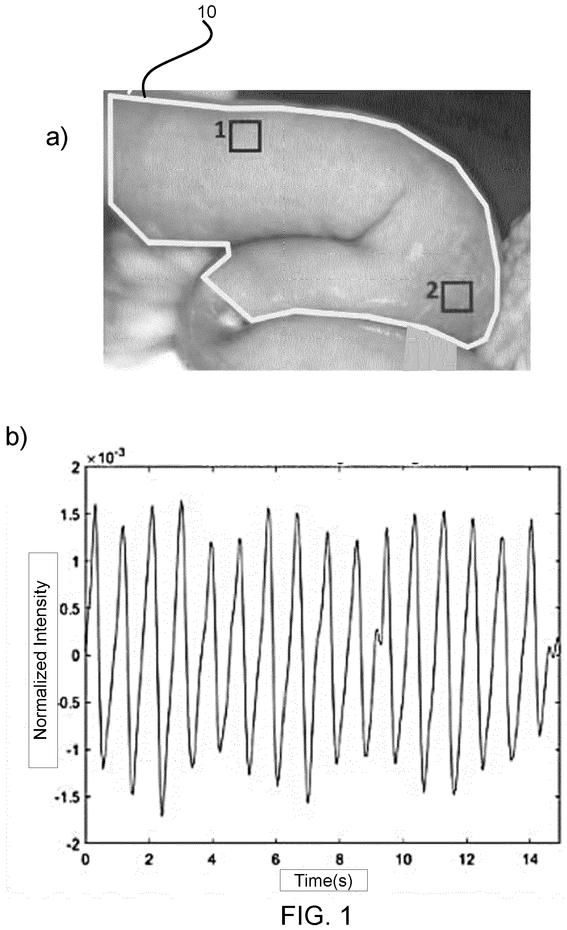

Figure 1 (a) shows a representation of a frame of a video acquired of the intestine. By spatial averaging the pixel values in the region of interest (ROI) 10, a signal can be derived from the video, modulated at the heart rate. This signal is shown in Figure 1 (b) as a normalized intensity of the PPG signal versus time.

The PPG signal of Figure 1 (b) represents an average for the whole region of interest.

However, separate signals may also be captured from smaller regions of interest, shown as region 1 and region 2 in Figure 1 (a).

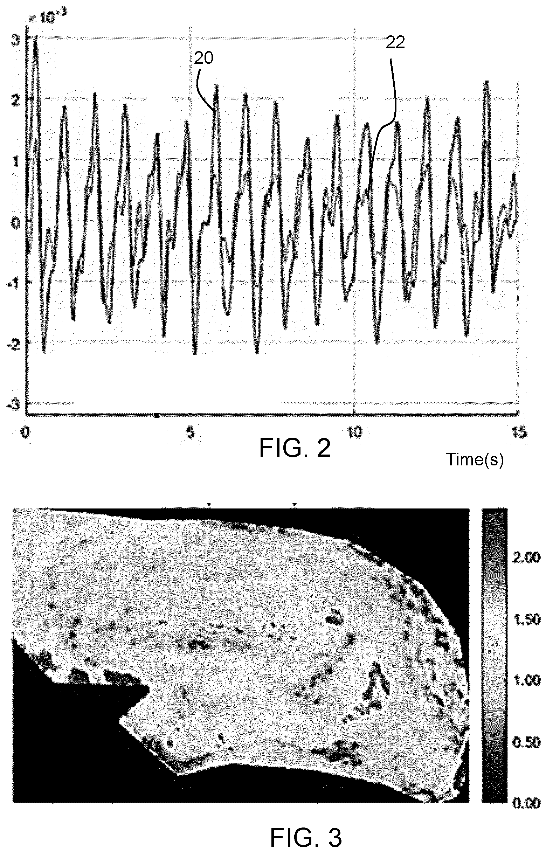

Figure 2 shows separate PPG signals, as a first signal 20 for the first region of interest and a second signal 22 for the second region of interest. The two PPG signals are modulated at the same heart rate frequency, but show a different amplitude. By extracting the amplitude from the PPG signal of each separate region of tissue, i.e. from each corresponding pixel of the captured video, a PPG perfusion map may be obtained, as shown in Figure 3.

Figure 3 is a black and white representation. However, the PPG perfusion may be color-coded, for example assigning a more red color to areas with higher perfusion and a more blue color to areas with lower perfusion. The PPG amplitude map thus represents the amplitude of the PPG signal per pixel and hence per location of the skin or other tissue being analyzed.

Additional valuable information may be obtained by analyzing the PPG signals.

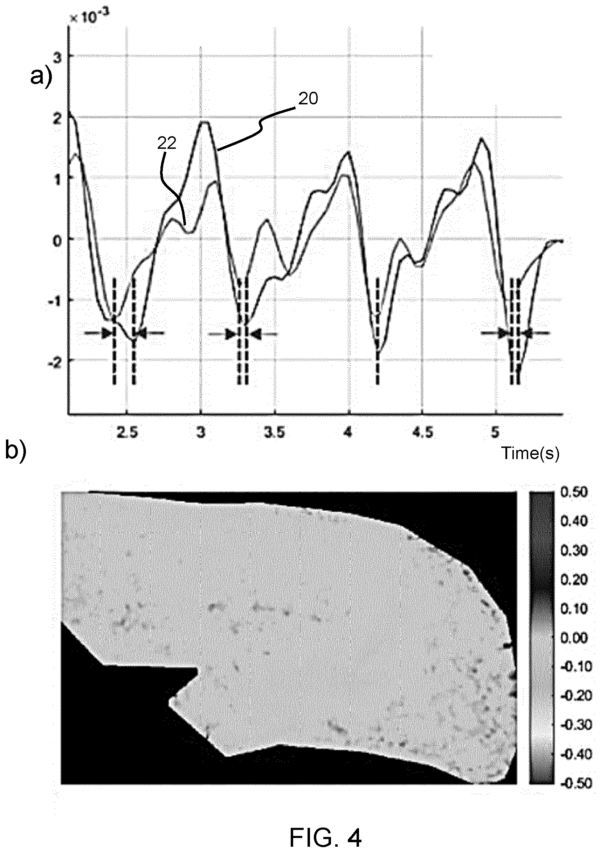

Figure 4 (a) shows a zoom-in of the PPG signals extracted from the two regions of interest from Figure 2. Even though the signals are extracted simultaneously from the same organ, the pulsation arrives slightly before in one area than in the other. There is a small delay in the blood pulsation arrival, due to small differences in the microcirculatory bed, such as the resistance and elasticity of the vessels, as well as different artery branches that supply the recorded tissues.

The delays of the PPG signals of each pixel with respect to a reference signal may be extracted and used for building a delay map. The delay map is shown in Figure 4.

Since the delay between the signals is not always constant but varies during the acquisition, an average delay is used between signals per pixel.

The average delay represents the average time delay between the PPG signal of each pixel and a reference PPG signal. In this way a signal in time is built. The length of the PPG signal should include at least a heartbeat cycle, so it is subject dependent.

The average delay is a value of delay assigned to each pixel. From the plot of Figure 4, it can be seen that there is a difference in time arrival between the peaks of the PPG signals. Therefore, for each pixel, the average of these delay (with respect to a reference signal) is assigned. At the end, the delay map is built, where each pixel contains the average delay between the PPG signal of that pixel and the reference PPG signal.

By acquiring a video stream, a series of images is obtained over time, for example with a frame rate of 20 frames per second. In order to compute the global PPG signal over time, the pixel values of the frame 1 are spatially averaged, so that the 2D set of pixels yields one value. The pixels of frame 2 are then averaged, and so on.

Eventually, a PPG signal is obtained over time (with the same length as the video that has been acquired), where each value of the signal is a spatial average of one corresponding frame of the video acquired. The image frames for example comprise 968x728 pixels, by way of example.

The PPG signal of each pixel is thus compared with the global PPG signal being used as a reference. The value assigned to each pixel "n" of the delay map thus represents the average delay between the PPG signal in the pixel "n" and the global PPG signal being used as a reference. Thus, the value of the delay for each pixel in the PPG delay map represents the average delay (in terms of average time shift) between the PPG signal of that pixel and the global PPG signal. The delays are for example computed by using the lock-in amplification algorithm.

The delay map thereby provides a measure of the average time delay between the PPG signal wave of each pixel and the reference PPG signal for at least one heartbeat cycle. The reference PPG signal is the global PPG signal of Figure 1 (b) extracted from the entire region on interest of the intestine.

Since the reference signal is extracted from the entire region of interest, a PPG signal from a given location of the image is likely to be in phase with the reference.

Similar to the map of the amplitude, Figure 4 (b) is a black and white representation. However, the PPG delay map may be color-coded. The Hue, Saturation, Value (HSV) color system is for example employed, since it works well with the periodicity of the PPG signal.

For extracting the amplitude maps and delay maps, a lock-in amplification method may be used.

Details on how to calculate the PPG maps using the lock-in amplification method can be found in:
(i) Lai M, Shan C, Ciuhu-Pijlman C, Izamis ML. Perfusion monitoring by contactless photoplethysmography imaging, 2019 IEEE 16th International Symposium on Biomedical Imaging (ISBI 2019) 2019 Apr 8 (pp. 1778-1782). IEEE; and
(ii) Lai M, Dicorato CS, de Wild M, Verbakel F, Shulepov S, Groen J, Notten M, Lucassen G, van Sambeek MR, Hendriks BH. Evaluation of a non-contact Photo-Plethysmographic Imaging (iPPG) system for peripheral arterial disease assessment, Medical Imaging 2021: Biomedical Applications in Molecular, Structural, and Functional Imaging 2021 Feb 15 (Vol. 11600, p. 116000F). International Society for Optics and Photonics.

Because of the great advantages that the remote PPG technology has shown for remotely and non-invasively assessing skin-level perfusion, PPG imaging can be translated to organ perfusion assessment for detecting the perfusion of the microvasculature tissue bed beneath the organ surface, without any modification to the current acquisition setup.

The invention relates to compensating for motion in the camera used to collect the images for PPG analysis and in the tissue being imaged.

Various motion stabilization algorithms are known. For example, the Kanade-Lucas-Tomasi (KLT) algorithm tracks a set of feature points across video frames and estimates geometric transformation between two frames. This algorithm allows to stabilize frames reducing motion in the region of interest, thus increasing the overlap between the same structures inside the region of interest in each frame. These transformations include rotation and translation, scaling, skewing and perspective distortion. All of them are implemented as linear transformation which are well-investigated in linear algebra.

A projective transformation is used to compensate for how perceived objects change when the view point of the observer changes. This transformation allows creating perspective distortion.

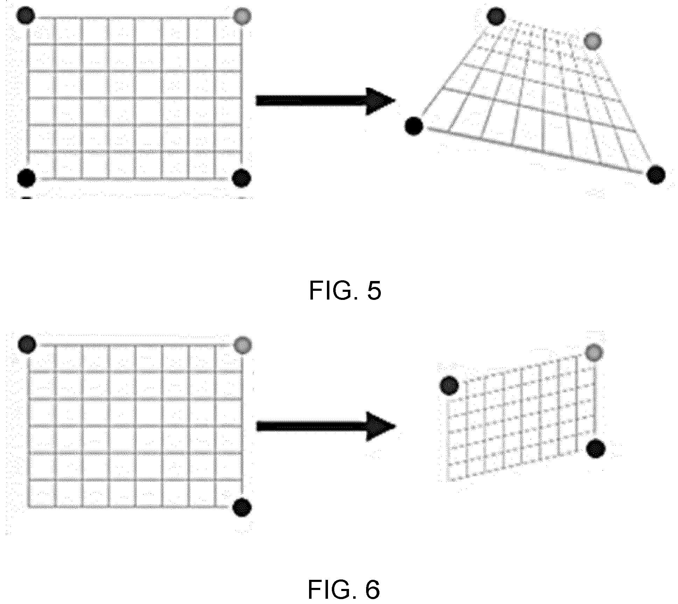

Figure 5 illustrates a projective transformation and Figure 6 illustrates an affine transformation for scaling, skewing and rotation.

The KLT algorithm mentioned above allows features to be extracted from an image (i.e. a 2D distribution of pixels), and the extracted features are used for stabilizing a region of interest, for example using projective or affine transformations. The feature points in the KLT algorithm are corners or borders in an image, easily identifiable across different images. The features points are typically in the form of isolated points, continuous curves or connected regions.

Also, motion stabilization algorithms implement linear transformations (e.g. projective transformations) which allow for translation, rotation, scaling of an image, while a registration with deformation gives enhanced results.

This invention makes use of a particular image registration approach between images to provide motion compensation.

Image registration is an optimization process about determining a spatial transformation that relates positions in one image (a reference image or a fixed image) to corresponding positions in one or more other images (a target image or moving image)

Image registration is used for the registration of medical images from the same image modality (mono-modal) on the same patient (intra-patient), to enable monitoring and quantifying a disease progression overtime, or evaluating the intra-operative organ deformation (e.g. brain deformation).

Image registration can also be used between different imaging modalities (multi-modal) on the same patient (intra-patient) for the correction of different patient position between scans, or even to allow the integration of anatomical and functional images (e.g. PET-CT scans).

Image registration methods can be classified as Rigid and Non-Rigid/Deformable methods. In the case of a rigid registration, the straightness of lines is preserved, as well as the point distances. This is typical of the transformations that include rotation, translation, zoom, and skew. This method works well for rigid anatomical structures, such as the bones. Non-rigid registration compensates for tissue deformation due to organ motion, which may occur in images taken at different time stamps.

The deformation is represented by a displacement vector of the image, which can be computed using several algorithms, such as the Free-Form Deformation (FFD) algorithm based on B-splines, or the Thin Plate Splines (TPS) algorithm, based on the Radial Basis Function (RBF).

As an example, for the Thin Plate Spline, an interpolation with deformation is achieved via a Spline plate with constraints (i.e. corresponding landmarks). The landmarks are selected and then matched in the two images, afterwards one of the images is warped such that the selected points of the second image match the points of the first image.

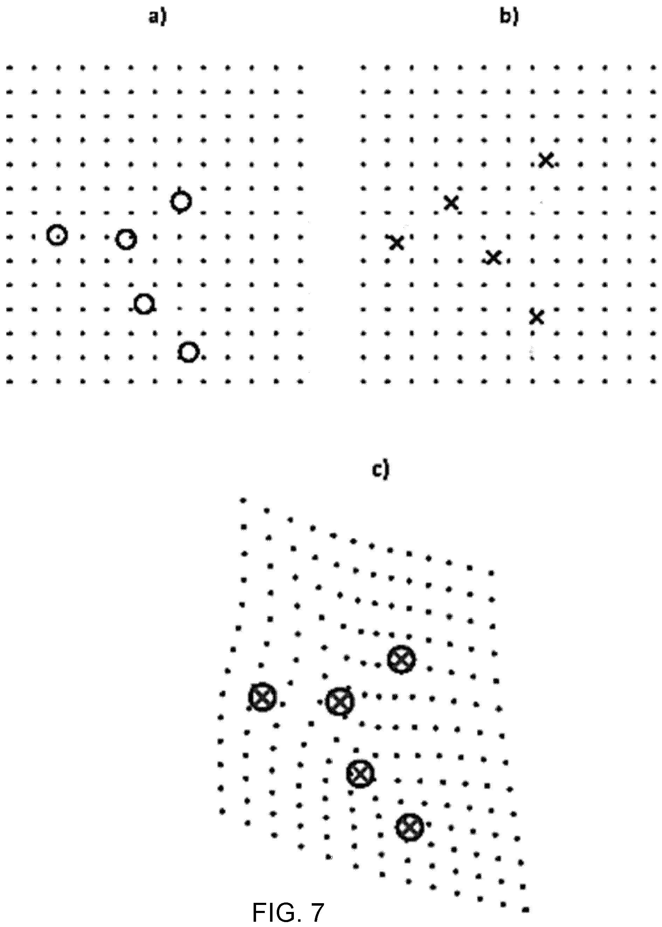

Figure 7 represents a registration approach with deformation using a Thin Plate Spline algorithm. Two images are shown in Figures 7(a) and 7(b), with corresponding anatomical landmarks shown. The second image of Figure 7(b) is deformed by interpolation such that the landmarks match the corresponding landmarks in Figure 7(a).

Details of this particular interpolation method may be found in Donato G, Belongie SJ. Approximation methods for thin plate spline mappings and principal warps. Department of Computer Science and Engineering, University of California, San Diego; 2003 Aug.

The required deformation of the image of Figure 7(b) is represented as Figure 7(c). The invention is based on the use of this type of registration with deformation to compensate for the motion and deformation of organs (e.g. organs in the abdominal area) due to respiration and heart beating in PPG imaging videos.

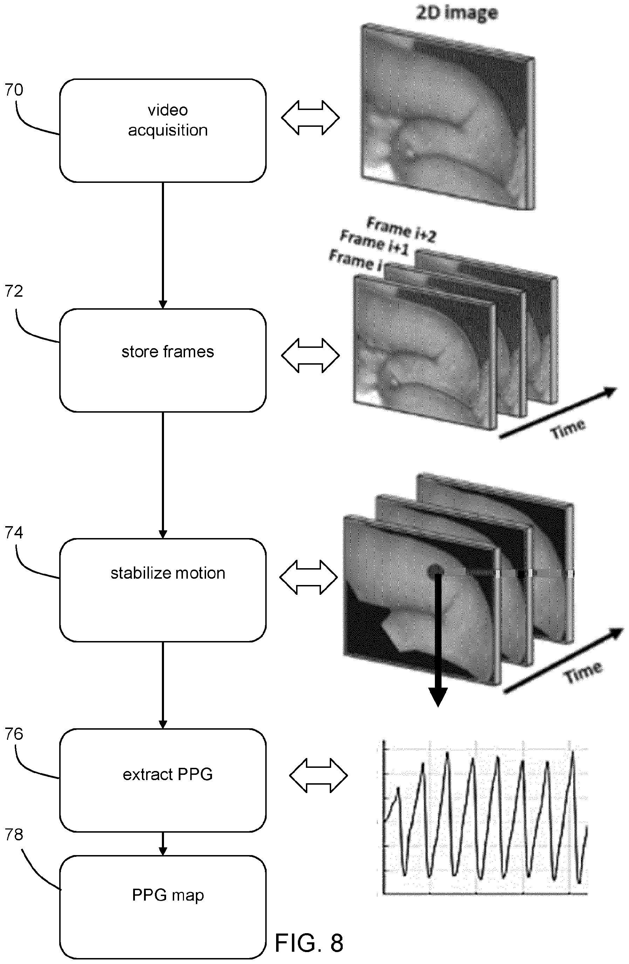

Figure 8 shows a generic approach for extracting PPG signals from pixels of a 2D endoscope and building a PPG perfusion map. Figure 8 shows a flow chart of method steps and also shows representations of the data being processed at the first four steps of the method.

Video acquisition takes place in step 70 with a standard 2D endoscope using a camera which may be at the distal end or at the proximal end (in which case the images are relayed to the camera along the endoscope). In step 72 multiple image frames are stored.

In step 74, the motion between frames is stabilized via software. For the motion stabilization, an algorithm is used to estimate the geometric transformation between frames. Afterwards, the PPG signal is extracted in step 76 from each pixel of the stabilized video.

Finally, a PPG map (PPG amplitude and/or PPG delay) may be built in step 78 using an algorithm, such as using lock-in amplification.

The invention relates to the way motion stabilization takes place in step 74 of Figure 8.

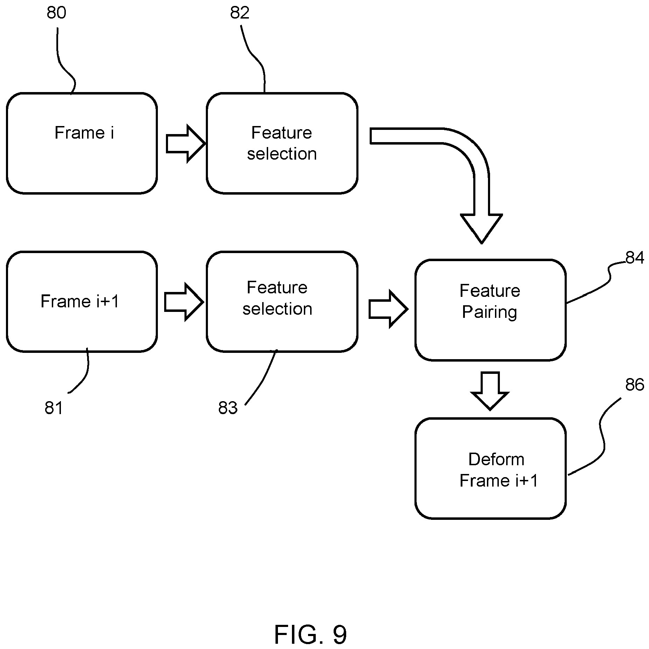

Figure 9 shows the motion stabilization approach to be employed within the general method of Figure 8. The motion stabilization involves registration with deformation of endoscopic videos, to enable PPG signals to be extracted from the image pixels so that a PPG map can be built. The motion stabilization involves operation of an algorithm that estimates the geometric transformation, with deformation, between frames (i.e. images) of the video stream. For example, a Free-Form Deformation (FFD) algorithm or a Thin Plate Splines (TPS) algorithm can be used.

In step 80, a first image (i.e. frame of video data) of a pair of images is received and in step 81 a second frame of the pair of images (i.e. a frame of video data at a different time) is received. For example, the second frame may be the next image frame, after the first frame, within the captured video sequence. However, the first frame may instead be a constant reference frame and the second frame is then any other frame of the video sequence.

In step 82, landmarks are identified in the first image and in step 83 landmarks are identified in the second image. Thus, landmarks are identified in each of the pair of images. The landmark identification is applied to all images to be used, and hence to multiple pairs of images. Thus, landmarks are identified in each of a set of the images which are to be motion-compensated with respect to each other.

The anatomical landmarks, such as a vessel, are used as features of a motion stabilization algorithm. The advantage is that the vessels can deform and stretch, together with the tissue, but still they remain connected among each other in their vascular tree conformation.

The overall set of images enable PPG signals to be derived, and they for example cover a time period of at least one heartbeat, for example at least 2 seconds.

For the pair of images shown, and more generally for multiple pairs of the images of the set, the landmarks are paired in step 84 between the first image of the pair and the second image of the pair.

The second image of the pair is then deformed in step 86 to match the landmarks in that second image of the pair with the landmarks in the first image of the pair. This provides motion compensation between the first and second images. As explained above, this involves interpolation and warping such that the selected landmarks of the second image match the landmarks of the first image.

A remote PPG signal can then be obtained from the set of the motion-compensated images, as shown in step 76 in Figure 8. A PPG signal may be obtained from any image pixel, or by averaging one or more groups of image pixels or by averaging all pixels of the image.

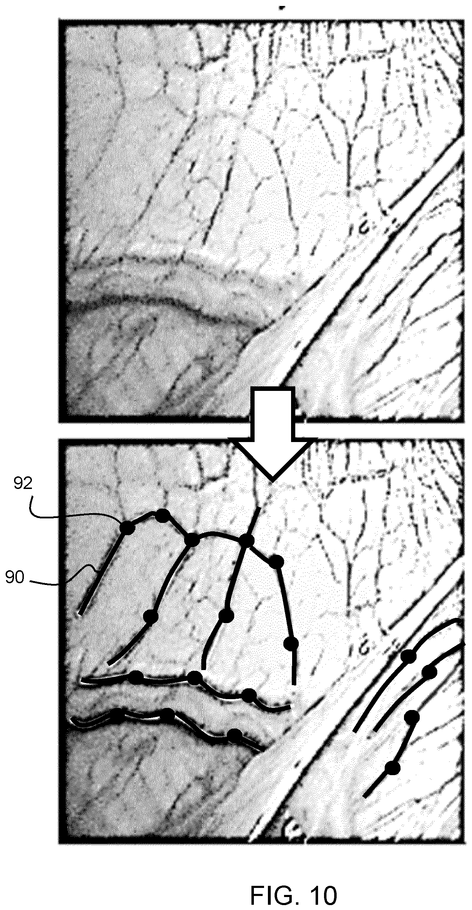

The landmarks are any identifiable anatomical features. Figure 10 shows an example of landmarks in an endoscopic video frame.

The top image shows an endoscopic video frame extracted from a video sequence. As an example, the microvasculature of the capillaries or arteries and veins can be used for the stabilization. The length of the entire vessel 90 can be used, or just some feature points 92 may be used such as the branching of the vessels. These landmarks could be selected either manually, or via semi-automatic and automatic algorithms.

The same area is recorded in sequential frames of the captured video, but because of the deformation of the tissue, the anatomical structures will have different positions and even shapes between the multiple images. The identified landmarks are then used to compensate for the deformation between images.

This invention can be used for the stabilization of 2D endoscopic videos acquired during minimally invasive surgery. The same method can be also used for the stabilization of areas outside the body (e.g. hands, feet) using stereo cameras systems.

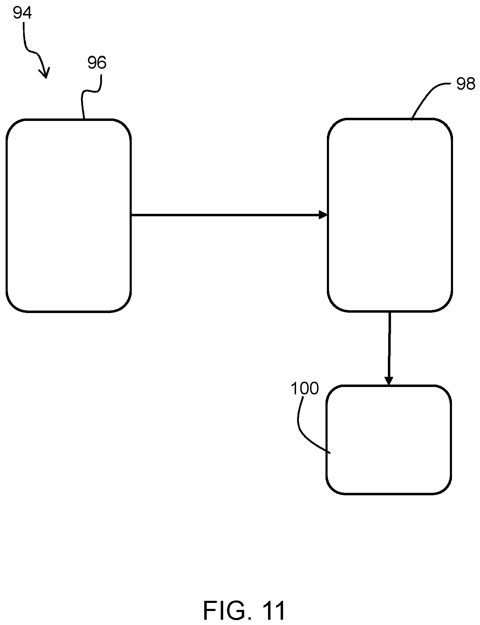

Figure 11 shows PPG imaging system 94 for processing images 96 to derive remote PPG signals. The system comprises a processor 98 adapted to receive images of a region of interest, identify landmarks in each of a set of the images and then for pairs of images of the set pair the landmarks between a first image of the pair and the second image of the pair and deform the second image of the pair to match the landmarks in that second image of the pair with the landmarks in the first image of the pair. This provides motion compensation between said pair of images. A remote PPG signal (or indeed set of PPG signals) can then be derived from a set of the motion-compensated images.

The processor 98 controls a display 100 to display the set of PPG signals, for example as a perfusion map and/or a delay map and/or a set of PPG waveforms.

Remote PPG sensing may be performed using a standard image sensor, which receives reflected ambient light.

However, there is also the option of using hyperspectral imaging. Hyperspectral imaging (HSI) is an emerging imaging modality for medical applications that offers great potential for noninvasive disease diagnosis and surgical guidance. The objective of hyperspectral imaging is to collect a three-dimensional dataset of spatial and spectral information, known as hypercube.

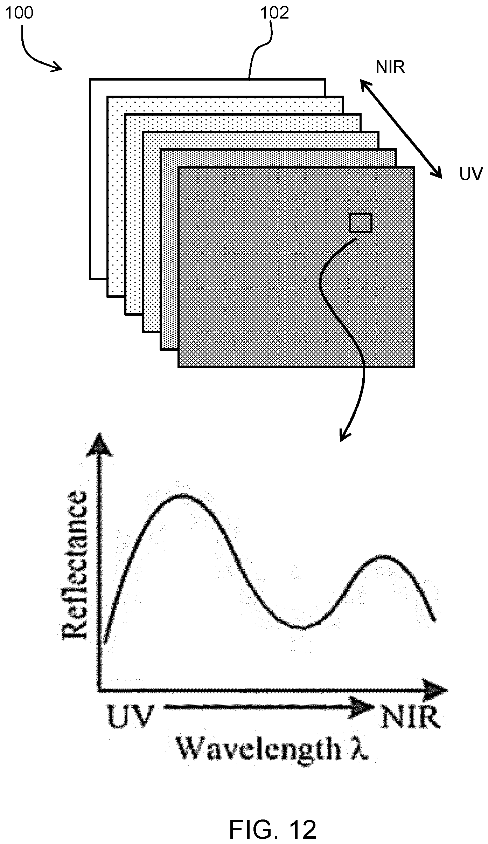

As shown in Figure 12, the hypercube is three-dimensional dataset 100 comprising twodimensional images 102 at each of a set of wavelengths. Figure 10 also shows a reflectance curve (i.e. the spectral signature) of a pixel in each image.

The hyperspectral camera may be used for detecting specific features in the field of view. For example, blood vessels could be better extracted from the infra-red wavelengths. The spectra of specific tissue (e.g. fat, muscle, blood) could be extracted from the hyper/multispectral camera and used as a feature for stabilizing the image.

The landmarks may be identified in standard 2D images, so that the approach does not require 3D imaging.

The example above is based on natural anatomical features being used as the landmarks for image stabilization. However, other landmarks may be used. For example, various alterations to the tissue are often made during surgery such as making cuts, and applying staples or sutures. These alterations can be used as landmarks for the motion stabilization.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A PPG imaging system (94) for processing images to derive remote PPG signals, comprising a processor (90) adapted to:
(70) receive images of a region of interest;
(82,83) identify landmarks in each of a set of the images;
for pairs of images of the set:
(84) pair the landmarks between a first image of the pair and a second image of pair; and
(86) deform said second image of the pair to match the landmarks in that second image of the pair with the landmarks in the first image of the pair thereby providing motion compensation between said pair of images; and
(76) derive a remote PPG signal from a set of the motion-compensated images.

2. The system of claim 1, wherein the processor is adapted to derive a set of remote PPG signals comprising a PPG perfusion map based on PPG amplitude levels obtained from the motion-compensated images at a set of image locations.

3. The system of claim 2, wherein the processor is adapted to derive a set of remote PPG signals comprising a PPG delay map based on PPG relative delays between image locations.

4. The system of any one of claims 1 to 3, wherein the images comprise 2D camera images.

5. The system of any one of claims 1 to 3, wherein the images comprise hyperspectral camera images.

6. The system of any one of claims 1 to 5, wherein the landmarks comprise a pattern of a microvasculature visible at the surface of the region of interest.

7. The system of claim 6, wherein the pattern of microvasculature is encoded as branch points and/or vessel lengths.

8. The system of any one of claims 1 to 7, wherein the landmarks comprise applied surgical features such as staples or sutures.

9. The system of any one of claims 1 to 8, wherein the processor is adapted to deform the second image of the pair by using an algorithm that combines image registration and deformation, such as a Free Form Deformation algorithm or a Thin Plate Spines algorithm.

10. The system of any one of claims 1 to 9, further comprising a 2D camera for capturing the images of the region of interest.

11. The system of claim 10, further comprising an endoscope, wherein the 2D camera is mounted at a distal end of the endoscope or at the proximal end of the endoscope.

12. A method for processing images to derive remote PPG signals, comprising:
(70) receiving images of a region of interest;
(82,83) identifying landmarks in each of a set of the images;
for pairs of the images of the set:
(84) pairing the landmarks between a first image of the pair and a second image of the pair; and
(86) deforming said second image of the pair to match the landmarks in that second image of the pair with the landmarks in the first image of the pair thereby providing motion compensation between said first and second images; and
(76) deriving a remote PPG signal from a set of the motion-compensated images.

13. The method of claim 12, comprising:
deriving a set of remote PPG signals as a PPG perfusion map based on PPG amplitude levels obtained from the images at a set of image locations; and/or
deriving a set of remote PPG signals as a PPG delay map based on PPG relative delays between image locations.

14. A computer program comprising computer program code means which is adapted, when said program is run on a computer, to implement the method of any one of claims 12 to 13.

15. A processor which is programed with the computer program of claim 14.
